# FASCICULE DE BREVET EUROPEEN

(11) **EP 0 791 373 B1**
(45) Date de publication et mention de la délivrance du brevet: **14.06.2000**
(21) Numéro de dépôt: 97400277.6
(22) Date de dépôt: 07.02.1997
(51) Int. Cl.: G06F 19/00, A61N 1/372, G05B 19/042

(54) **Dispositif médical implantable actif et son programmateur externe à mise à jour automatique du logiciel**
Aktive implantierbare medische Vorrichtung und dazugehörende externe Programmiereinrichtung mit automatischer Aktualisierung von integrierter Software
Active implantable medical device and associated external programmer with automatic updating of embedded software

(30) Priorité: 08.02.1996 FR 9601564
(43) Date de publication de la demande: 27.08.1997
(73) Titulaire: ELA MEDICAL (Société anonyme), F-92541 Montrouge (FR)
(72) Inventeur: Faisandier, Yves, 75014 - Paris (FR)
(74) Mandataire: Dupuis-Latour, Dominique

(56) Documents cités:
- WO-A-92/03776
- US-A- 4 513 743
- US-A- 4 712 179
- US-A- 5 036 869
- US-A- 5 321 618
- PROCEEDINGS OF THE ANNUAL INTERNATIONAL CONFERENCE OF THE ENGINEERING IN MEDICINE AND BIOLOGY SOCIETY, PARIS, OCT. 29 - NOV. 1, 1992 DOJAT M ET AL: 'AN EXTENDABLE KNOWLEDGE-BASED SYSTEM FOR THE CONTROL OF MECHANICAL VENTILATION' vol. 14, PART 3, 29 Octobre 1992, MORUCCI J P;PLONSEY R; COATRIEUX J L; SWAMY LAXMINATAYAN, page 920/921, XP000480685
- MICROPROCESSORS AND MICROSYSTEMS, vol. 18, no. 9, 1 Novembre 1994, pages 523-535, XP000483416 DAMJAN ZAZULA ET AL: "COMPUTER-ASSISTED EXERCISE ECG ANALYSIS: REAL-TIME SCHEDULING WITHIN MS-DOS ON PCS"
- HEWLETT-PACKARD JOURNAL, vol. 42, no. 4, 1 Octobre 1991, pages 6-10, XP000362106 WESTERTEICHER C: "INTRODUCTION TO THE HP COMPONENT MONITORING SYSTEM"
- PROCEEDINGS OF THE ANNUAL INTERNATIONAL CONFERENCE OF THE ENGINEERING IN MEDICINE AND BIOLOGY SOCIETY, PARIS, OCT. 29 - NOV. 1, 1992 HERRERA-BENDEZU L G ET AL: 'INTEGRATED CARDIAC WORKSTATION FOR IMAGE AND SIGNAL PROCESSING' vol. 14, 29 Octobre 1992, MORUCCI J P;PLONSEY R; COATRIEUX J L; SWAMY LAXMINARAYAN, pages 2720 - 2721, XP000347089
- PROCEEDINGS OF THE ANNUAL SYMPOSIUM ON COMPUTER BASED MEDICAL SYSTE, ANN ARBOR, JUNE 13 - 16, 1993, no. SYMP. 6, 13 Juin 1993, INSTITUTE OF ELECTRICAL AND ELECTRONICS ENGINEERS, pages 253-257, XP000416020 SNYDER A J ET AL: "A SECURE COMMUNICATIONS AND STATUS REPORTING PROTOCOL FOR IMPLANTED DEVICES"
- IEEE ENGINEERING IN MEDICINE AND BIOLOGY MAGAZINE, IEEE PRESS, vol. 12, no. 4, Décembre 1993, NEW YORK, USA, pages 69-81, XP002021620 SUKUWAARA T. I. ET AL: "object oriented implementation of an architecture for patient monitoring"

## Description

La présente invention concerne les dispositifs médicaux implantables actifs et les appareils appelés "programmateurs" destinés à échanger des données avec ces derniers.

L'invention sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des Communautés Européennes, définition qui inclut, outre les stimulateurs cardiaques, les défibrillateurs et/ou cardioverteurs, les pompes de diffusion de substances médicales, les implants cochléaires, les capteurs biologiques implantés, etc.

Ces dispositifs (ci-après désignés simplement "implants"), une fois mis en place, sont programmés de l'extérieur au moyen d'une console appelée "programmateur" généralement constitué d'un micro-ordinateur équipé d'une tête de programmation placée en regard du site de l'implant. Le programmateur peut lire par voie électromagnétique ("télémétrie") des données conservées dans l'implant (par exemple des paramètres de fonctionnement, des signaux biologiques qui ont été enregistrés, etc.) et, d'autre part, envoyer éventuellement certaines données vers cet implant afin de pouvoir le "reprogrammer", c'est-à-dire en modifier les paramètres de fonctionnement.

Ces programmateurs sont tous spécifiques à un type d'implant, ou à une famille d'implants d'un même fabricant, de sorte que la mise sur le marché d'un nouvel implant impose généralement au fabricant une mise à jour correspondante des programmateurs. En outre, même en l'absence de mise sur le marché d'un nouvel implant, des perfectionnements sont régulièrement apportés, qui peuvent conduire à une mise à jour du logiciel du programmateur.

Ces mises à jour sont particulièrement délicates.

En effet, pour des raisons évidentes de sécurité, elles doivent être réalisées selon un protocole très strict : visite dans chaque centre, changement du programme par une personne qualifiée, récupération des anciens programmes, rédaction et signature de documents de certification, etc. Ces procédures sont lourdes et onéreuses, de sorte que l'on évite de les appliquer trop souvent (par exemple à l'occasion de modifications mineures du logiciel). En outre, les procédures de sécurité, pour complètes qu'elles soient, n'éliminent pas totalement le risque d'une absence de mise à jour (d'un programmateur qui ne serait pas localisé par le fabricant), ni le problème des périodes de transition, et surtout l'utilisation inappropriée d'une ancienne version du logiciel.

L'un des buts de l'invention est de remédier à ces difficultés, en proposant un nouveau concept de programmateur dont le logiciel puisse être mis à jour de façon entièrement automatique, sans intervention humaine et en s'affranchissant donc des lourdeurs, des coûts et des risques liés aux procédures actuelles.

L'invention implique la mise en oeuvre de la notion d"'objet" au sens logiciel du terme, c'est-à-dire d'un ensemble d'éléments, au sens le plus large du terme (données, constructeurs, destructeurs et méthodes), permettant de gérer et d'afficher des données de manière très structurée. En particulier, l'accès aux données ne s'effectue en général qu'au travers de méthodes propres à l'objet, ce qui permet de protéger ce derniers contre les écritures hors limite et de rendre le logiciel particulièrement fiable. Le concept d"'héritage" facilite également la conception du logiciel, un objet pouvant hériter d'un autre, ce qui lui permet d'utiliser directement les méthodes de ce dernier.

Des exemples d'objets logiciels et de leurs applications au domaine des stimulateurs cardiaques seront donnés plus bas.

De façon générale, on pourra se référer à la littérature sur ce sujet, par exemple l'ouvrage de Max Bouché, *La démarche objet - Concepts et outils,* AFNOR, 1994, ainsi que la documentation des langages de programmation "orientés objet" tels que le C++ qui est un langage typique de programmation orientée objet.

Essentiellement, l'idée de l'invention consiste à (i) stocker dans l'implant lui-même les objets logiciels permettant de définir l'affichage, le traitement et la programmation de ses données par un programmateur externe, (ii) faire comparer par le programmateur la liste des objets que ce programmateur contient à la liste des objets que l'implant contient, et (iii) télécharger de l'implant vers le programmateur les objets manquants ou plus récents pour mettre à jour le logiciel du programmateur.

Cette manière de procéder, que l'on décrira plus en détail par la suite, procure quatre avantages majeurs par rapport à l'art antérieur ;
1° sécurité : tout programmateur équipé d'un noyau de base minimal, c'est-à-dire des objets permettant d'établir la communication avec un implant et de gérer les liaisons entre objets, pourra lire tout implant, même si celui-ci a été développé bien plus tard que le programmateur. L'opérateur ne peut donc plus se retrouver dans l'impossibilité de lire et reprogrammer des fonctions d'un modèle récent d'implant, dès lors qu'il dispose d'un programmateur compatible, même ancien. De plus, chaque paramètre dispose de son propre mode de gestion, défini par un logiciel dans l'implant lui-même, et les contrôles de saisie de ces paramètres par l'opérateur ne peuvent pas être faux, puisqu'ils ne sont plus contrôlés par un objet préexistant du programmateur mais par un objet issu de l'implant lui-même.
2° économie : les frais de mise à jour des programmateurs sont considérablement réduits et ne s'imposent que pour les changements importants de matériel (nouvelles générations de programmateurs). On évite ainsi tous les frais des procédures de sécurité classiques qui accompagnent ces changements : fabrication de disquettes et de guides de mise à jour, déplacement d'un opérateur qualifié sur chaque site, signature d'un registre attestant la mise à jour, récupération des anciennes disquettes, etc.
3° simplification de la programmation : l'évolution du logiciel du programmateur s'effectue automatiquement et de façon "transparente" pour l'utilisateur. Seul des fonctions très générales pourront compléter la gestion des objets de base, et la liste des objets fournis au départ sera complétée par les objets définis dans chaque nouvel implant.
4° standardisation : la souplesse d'emploi de l'invention permet maintenant d'envisager la création d'un standard permettant à tout programmateur de lire n'importe quel implant, quel que soit son modèle ou son fabricant. Chaque fabricant pourra définir l'aspect graphique de son programmateur, les possibilités de gestion des données, les calculs supplémentaires, etc. de façon à garder une certaine originalité, mais, les objets de base étant définis de manière identique, la lecture d'un implant "étranger" devient parfaitement possible. On peut ainsi envisager la réalisation de programmateurs universels, ce qui était exclu jusqu'à présent non seulement pour des raisons de coûts et de complexité, mais également pour des raisons de sécurité (risques d'incompatibilités imprévues).

Plus précisément, on peut définir l'invention par un ensemble comprenant, d'une part, un implant du type dispositif médical implantable actif et, d'autre part, un programmateur externe d'implant, ensemble caractérisé en ce que :
- le programmateur comporte une mémoire contenant un logiciel structuré de façon modulaire du type orienté object composé d'un ensemble de modules logiciels du type object,
- l'implant comporte une mémoire contenant, d'une part, des données de paramétrage du fonctionnement de l'implant et, d'autre part, un ensemble d'objets logiciels nécessaires au fonctionnement d'un programmateur en relation avec lesdites données de paramétrage de l'implant,
- le programmateur et l'implant comportent des moyens aptes à établir entre eux une liaison bidirectionnelle d'échange d'informations permettant notamment le transfert sélectif de tout ou partie desdites données et desdits objets de l'implant qui sont manquant dans le logiciel du programmateur vers le programmateur, et
- le programmateur comporte des moyens de mise à jour, aptes à commander le téléchargement depuis l'implant d'au moins une partie desdits objets contenus dans la mémoire de ce dernier et à ajouter et/ou substituer ces objets à ceux du logiciel du programmateur.

Avantageusement, les moyens de mise à jour du programmateur comportent des moyens aptes à établir, par lecture de la mémoire de l'implant, une liste des objets qui s'y trouvent avec leur version respective ; des moyens aptes à comparer cette liste à celle des objets du logiciel du programmateur ; des moyens aptes à commander le téléchargement depuis l'implant des objets absents du logiciel du programmateur et ajouter ces objets au logiciel ; et/ou des moyens aptes à commander le téléchargement depuis l'implant des objets présents dans le logiciel du programmateur mais dont la version est antérieure à celle qui se trouve dans l'implant, et à remplacer ces objets dans le logiciel.

L'invention couvre également, en tant que produit indépendant, l'implant ou bien le programmateur d'implant présentant les caractéristiques précitées.

On va maintenant décrire un exemple de mise en oeuvre de l'invention.

Comme on l'a indiqué plus haut, le logiciel du programmateur, tout comme celui de l'implant, est organisé en "objets logiciels".

Dans le cas d'un stimulateur cardiaque, on peut représenter par un objet chaque élément du stimulateur qui est programmable ou simplement affichable sur l'écran d'un programmateur.

Ainsi, une courbe donnant la fréquence cardiaque en fonction du temps peut être représentée par un objet particulier *Fréquence-cardiaque,* qui hérite d'un objet plus général que l'on peut appeler *Courbe* et comprenant une suite de données correspondant à une courbe, avec son format, ses échelles en abscisse et en ordonnée, son titre, une méthode d'affichage, une méthode d'impression, etc.

Le programmateur, quant à lui, dispose d'un objet *Gestionnaire-de-liaison* qui peut appeler les données correspondant à la fréquence cardiaque stockées dans le stimulateur en appliquant les protocoles de transfert, compression/décompression de données, etc. nécessaires. Le programmateur pourra ensuite afficher une fenêtre présentant ces données, à l'aide de l'objet *Courbe* permettant toutes les fonctions courantes : zoom, défilement dans la fenêtre, mesures à l'aide de curseurs, etc.

Le concept de l'invention repose sur trois hypothèses fondamentales :
- le logiciel du programmateur est "ouvert", c'est-à-dire qu'il peut s'enrichir en recevant et incorporant de nouveaux objets concernant l'affichage des données, le traitement de ces données et la programmation de l'implant. Ces objets peuvent provenir soit de l'implant (c'est la caractéristique de l'invention) soit d'une disquette ou de tout autre support informatique de manière en elle-même classique, par exemple pour le chargement préalable de l'objet *Gestionnaire-de-liaison.*
- l'implant stocke en lui-même tous les objets permettant de définir l'affichage, le traitement et la programmation des données contenues dans cet implant. On verra plus loin que la taille de ces objets peut être relativement réduite, dans la mesure où les objets dont ils héritent (par exemple l'objet *Courbe* définissant l'affichage et la gestion d'une courbe en général par le programmateur) sont déjà présents dans le programmateur.
- les versions des objets sont datées (par exemple par un numéro de version) et toute version plus récente doit remplacer dans le programmateur une version plus ancienne. Les développements des objets s'effectuent en imposant une compatibilité ascendante, c'est-à-dire qu'une version plus récente reste compatible avec tous les échanges de données relatives aux versions plus anciennes.

Au début de l'échange de données entre implant et programmateur, ce dernier appelle systématiquement un bloc appelé *Liste-des-objets* qui est un répertoire des objets disponibles dans l'implant avec leur version. Il compare alors les codes de ces objets avec ceux qu'il connaît (liste des objets disponibles dans le logiciel du programmateur, gérée dynamiquement et sauvegardée dans un fichier de configuration). Si un ou plusieurs de ces objets est inconnu du programmateur, il appelle alors par le *Gestionnaire-de-liaison* une procédure de téléchargement de ces objets afin de compléter son logiciel et l'adapter ainsi à l'implant. Cette procédure de téléchargement et de mise à jour consiste, pour chaque objet inconnu, à faire fonctionner le *Gestionnaire-de-liaison,* dont hérite cet objet ; le *Gestionnaire-de-liaison* envoie le numéro de l'objet au stimulateur qui, en retour, lui renvoie un bloc d'informations correspondant.

Il en est de même pour les objets qui, bien que présents dans le logiciel du programmateur, correspondent à une version plus ancienne que celle contenue dans l'implant.

Après cette phase, le programmateur peut appeler toutes les données contenues dans l'implant, afficher les résultats souhaités, reprogrammer l'implant, etc.

Ainsi, dans le cas d'un implant disposant de l'objet *Fréquence-cardiaque* lu par un programmateur ne le connaissant pas, celui-ci repère l'objet dans la *Liste-des-objets* fournie par le stimulateur. Il demande le transfert de l'objet et l'ajoute à ses bases de programme, l'appel au constructeur de l'objet permettant de réserver une zone mémoire pour recevoir ultérieurement la courbe de fréquence, la sélection d'une fenêtre d'affichage et l'initialisation de diverses variables. Le code de l'objet *Fréquence-cardiaque* vient alors s'ajouter à la liste dynamique des objets disponibles dans le logiciel du programmateur.

Lorsque l'opérateur veut afficher la courbe, il appelle sur le programmateur la fenêtre correspondante. Le programme teste alors la présence de la courbe en mémoire et, dans la négative, il engage une lecture des données de fréquence cardiaque mémorisées dans le stimulateur. La courbe s'affiche ensuite dans une fenêtre disposant des commandes classiques d'ajustage (gain, zoom, défilement), de mesure (curseurs) et éventuellement d'impression, ces diverses fonctions étant gérées par l'objet général *Courbe* déjà présent dans le programmateur.

On va maintenant décrire deux exemples détaillés d'objets de stimulateur : un objet *Fréquence-nominale* (valeur fixe programmée) et un objet *Fréquence-respiratoire* (donnée évolutive au cours du temps).

La description des objets peut être réalisée sous plusieurs formes, que l'on peut résumer à trois cas : code machine, code précompilé et métalangage :
- Le code machine est trop lié au processeur pour permettre un usage à long terme.
- Le code précompilé (comme l'ancien "P-code") est extrêmement performant, pouvant décrire toutes les données et les méthodes des nouveaux objets. Il reste par contre d'un volume important, mal adapté aux implants de faible capacité.
- Le métalangage ne fait que préciser la valeur des variables appartenant à des objets parents prédéfinis par le programmateur. Le volume des données est de ce fait extrêmement réduit. La plupart des données d'un implant peuvent se contenter d'une description sous forme d'un métalangage, mais le recours à un code précompilé doit pouvoir rester accessible pour l'intégration d'objets originaux.

Dans l'exemple décrit ici, le langage choisi est un métalangage, qui procure une taille réduite des objets et une indépendance totale au langage de programmation. Par contre, il ne peut faire que référence aux objets de base du programmateur, sans pouvoir les faire directement progresser. Les limites ne sont atteintes que lorsqu'un concept complètement nouveau ne pourra être intégré par ceux-ci, de sorte que le développement de ces objets de base doit être extrêmement bien réalisé pour éviter la survenue trop fréquente de ce genre d'incident qui imposerait un mise à jour traditionnelle des logiciels des programmateurs.

### Objet Fréquence-nominale

La fréquence nominale, qui est un paramètre essentiel de tout stimulateur cardiaque, peut être représenté par un objet décrit de la manière suivante :
- numéro d'objet : code unique affecté à l'objet, par exemple sur huit caractères (deux lettres pour le fabricant, quatre lettres pour l'objet et deux chiffres pour le numéro de version), qui permet de choisir un code à la fois univoque et facilement déchiffrable.
- objet ancêtre : un numéro d'objet fournit le code de l'objet ancêtre, qui réside dans ce cas dans le programmateur dès l'origine et qui permet de gérer tous les procédés d'affichage, de saisie, d'impression et de stockage d'une valeur numérique. Cet objet hérite, bien sûr, au minimum, d'un objet plus général qui est le *Gestionnaire-de-liaison.* Comme il s'agit d'une programmation par pas prédéfinis, les valeurs sont codées en nombre de pas. Cette donnée occupe cinq caractères.
Les données suivantes paramètrent l'objet ancêtre :
- phrase descriptive (100 caractères) accompagnant la valeur numérique à l'affichage (par exemple vingt caractères, soit cent caractères au total pour cinq langues différentes).
- unité de mesure (10 caractères).
- valeur du pas, du minimum, du maximum et de la valeur nominale (16 caractères) : les limites résident dans l'objet ancêtre afin d'empêcher la saisie d'une valeur numérique hors limite. Ces limites sont mises à jour par le maximum et le minimum et la valeur nominale remplace la valeur présente dans l'objet lors de l'appel d'une méthode de "mise au nominal" dont héritent tous les objets programmables. Le pas peut être défini en valeur constante, logarithmique ou inverse de façon à répondre aux divers cas de figures présents dans les stimulateurs.
- fenêtre de référence (5 caractères) : chaque donnée doit être affichée dans l'un des écrans du programmateur, qui peut contenir lui-même une à plusieurs fenêtres. Chaque fenêtre est définie par le stimulateur avec au moins un titre, un format (une colonne, deux colonnes, etc.) et des actions que l'on peut mener (déplacement, appel à une lecture momentanée ou continue du stimulateur, menu local, etc.).
- position dans la fenêtre de référence (5 caractères) : colonne, hauteur, etc., qui peuvent se résumer sous la forme d'un nombre à cinq chiffres : le premier donne la colonne, les trois suivants un ordre de priorité par rapport aux autres ; le noyau de gestion d'écran les place alors de haut en bas par ordre de priorité.
L'ensemble de ces données occupent donc 56 caractères en mode monolingue et 136 caractères en mode multilingue.

### Objet Fréquence-respiratoire

Dans le cas d'un stimulateur analysant la fréquence respiratoire, celle-ci peut être stockée sur des périodes de plusieurs heures, plusieurs jours et même plusieurs mois. L'objet peut être décrit en métalangage de la façon suivante :
- numéro d'objet (8 caractères) : voir ci-dessus.
- objet ancêtre. (5 caractères) : Il s'agit d'un générateur de courbe en fit) possédant une échelle verticale et une échelle horizontale, un format de données et un mode d'affichage (trait, minimum-maximum, etc.), des méthodes de changement d'échelle, d'affichage et d'impression. Cet objet fait partie des objets résidant dans le programmateur.
Les données suivantes paramètrent l'objet ancêtre :
- phrase descriptive (20 caractères par langue, soit 100 caractères en mode multilingue) : voir ci-dessus.
- unité de mesure (10 caractères).
- format des données (5 caractères) : suite d'entiers (8, 16, 24 ou 32 bits), de réels, etc. Ce format est lui-même un objet résidant qui hérite du *Gestionnaire-de-liaison* afin de le rendre capable de gérer le transfert des données entre stimulateur et programmateur.
- période de temps séparant chaque donnée, (4 caractères): période en millisecondes, codée par un mot de quatre octets permettant ainsi une dynamique de 1 ms à 1137 heures.
- description de l'échelle verticale (12 caractères) : valeur basse, valeur haute, pas.
- fenêtre de référence (5 caractères) : comme dans le cas de la fréquence nominale.
- position dans la fenêtre de référence (5 caractères) : comme pour la fréquence nominale, l'affichage du graphique se positionne selon un certain ordre de priorité ; le nombre de colonnes et le nombre d'éléments affichés sont pris en compte par la fenêtre pour accorder à chacun le maximum de place disponible : ainsi, une fenêtre à une colonne ne contenant qu'une courbe affiche celle-ci sur toute la surface disponible, une fenêtre à deux colonnes et quatre courbes affiche chacune sur un quart de la surface totale, etc.

L'ensemble de ces données occupe donc 74 caractères en mode monolingue et 154 caractères en mode multilingue.

On peut décrire ainsi de la même manière tous les paramètres de fonctionnement du stimulateur.

Dans le cas d'un stimulateur cardiaque relativement compliqué, comprenant 20 courbes en mémoire et 50 paramètres programmables, la taille totale des objets est ainsi, en mode multilingue, de 50 x 136 = 6800 caractères pour les paramètres et 20 x 154 = 3080 caractères pour les courbes, soit un total de 9880 caractères, auxquels il faut ajouter le bloc *Liste-des-objets* qui sera transmis au programmateur préalablement à toute mise à jour, soit 70 x 8 = 540 octets.

La taille totale réservée au stockage des objets dans le stimulateur n'atteint donc qu'à peine 10 Ko environ dans les cas les plus complexes (par exemple un stimulateur DDD asservi de dernière génération) et ces éléments peuvent être aisément introduits dans une puce de type ROM du stimulateur, sans entraîner de surconsommation tant que cette puce n'est pas activée.

## Revendications

1. Un ensemble comprenant, d'une part, un implant du type dispositif médical implantable actif et, d'autre part, un programmateur externe d'implant, caractérisé en ce que :
- le programmateur comporte une mémoire contenant un logiciel structuré de façon modulaire du type orienté objet composé d'un ensemble de modules logiciels du type objet,
- l'implant comporte une mémoire contenant, d'une part, des données de paramétrage du fonctionnement de l'implant et, d'autre part, un ensemble d'objets logiciels nécessaires au fonctionnement d'un programmateur en relation avec lesdites données de paramétrage de l'implant,
- le programmateur et l'implant comportent des moyens aptes à établir entre eux une liaison bidirectionnelle d'échange d'informations permettant notamment le transfert sélectif de tout ou partie desdites données et desdits objets de l'implant qui sont manquants dans le logiciel du programmateur, vers le programmateur, et
- le programmateur comporte des moyens de mise à jour, aptes à commander le téléchargement depuis l'implant d'au moins une partie desdits objets contenus dans la mémoire de ce dernier et à ajouter et/ou substituer ces objets à ceux du logiciel du programmateur.

2. L'ensemble de la revendication 1, dans lequel les moyens de mise à jour du programmateur comprennent :
. des moyens aptes à établir, par lecture de la mémoire de l'implant, une liste des objets qui s'y trouvent avec leur version respective,
. des moyens aptes à comparer cette liste à celle des objets du logiciel du programmateur,
. des moyens aptes à commander le téléchargement depuis l'implant des objets absents du logiciel du programmateur et à ajouter ces objets au logiciel, et/ou
. des moyens aptes à commander le téléchargement depuis l'implant des objets présents dans le logiciel du programmateur mais dont la version est antérieure à celle qui se trouve dans l'implant, et à remplacer ces objets dans le logiciel.

3. Un ensemble selon la revendication 1, dans lequel lesdits objets logiciels comprennent chacun au moins un identifiant d'objet, un identifiant de version et un bloc d'instructions de programme.

4. Un implant du type dispositif médical implantable actif, destiné à coopérer avec le programmateur de l'ensemble selon la revendication 1, implant caractérisé en ce qu'il comporte :
- une mémoire contenant, d'une part, des données de paramétrage du fonctionnement de l'implant et, d'autre part, un ensemble d'objets logiciels nécessaires au fonctionnement d'un programmateur en relation avec lesdites données de paramétrage de l'implant, ces objets étant susceptibles d'être émis vers le programmateur pour compléter ou mettre à jour le logiciel de ce dernier par le transfert sélectif vers le programmateur de tout ou partie desdites données et desdits objets qui sont manquants dans le logiciel du programmateur.

5. L'implant de la revendication 4, dans lequel lesdits objets logiciels comprennent chacun au moins un identifiant d'objet, un identifiant de version et un bloc d'instructions de programme.

6. Un programmateur externe, destiné à coopérer avec l'implant de type dispositif médical implantable actif de l'ensemble selon la revendication 1, programmateur caractérisé en ce qu'il comporte :
- une mémoire contenant un logiciel structuré de façon modulaire du type orienté objet composé d'un ensemble de modules logiciels du type objet,
- des moyens aptes à établir avec l'implant une liaison bidirectionnelle d'échange d'informations permettant notamment le transfert sélectif, de l'implant vers le programmateur, de tout ou partie de données de paramétrage du fonctionnement de l'implant et d'objets logiciels nécessaires au fonctionnement d'un programmateur en relation avec lesdites données de paramétrage de l'implant qui sont manquants dans le logiciel du programmateur, et
- des moyens de mise à jour, aptes à commander le téléchargement depuis l'implant d'au moins une partie desdits objets contenus dans la mémoire de ce dernier et à ajouter et/ou substituer ces objets à ceux du logiciel du programmateur.

7. Le programmateur de la revendication 4, dans lequel les moyens de mise à jour comprennent :
. des moyens aptes à établir, par lecture de la mémoire de l'implant, une liste des objets qui s'y trouvent avec leur version respective,
. des moyens aptes à comparer cette liste à celle des objets du logiciel du programmateur,
. des moyens aptes à commander le téléchargement depuis l'implant des objets absents du logiciel du programmateur et à ajouter ces objets au logiciel, et/ou
. des moyens aptes à commander le téléchargement depuis l'implant des objets présents dans le logiciel du programmateur mais dont la version est antérieure à celle qui se trouve dans l'implant, et à remplacer ces objets dans le logiciel.

8. Le programmateur de la revendication 6, dans lequel lesdits objets logiciels comprennent chacun au moins un identifiant d'objet, un identifiant de version et un bloc d'instructions de programme.

## Patentansprüche

1. Gesamtheit, die zum einen ein Implantat vom Typ einer aktiven implantierbaren medizinischen Vorrichtung und zum anderen einen externen Programmierer für das Implantat aufweist, dadurch gekennzeichnet, daß:
- der Programmierer einen Speicher aufweist, der ein auf modulare Weise strukturiertes Programm vom objektorientierten Typ enthält, das aus einer Gesamtheit von Programmmodulen vom Objekttyp zusammengesetzt ist,
- das Implantat einen Speicher aufweist, der zum einen Daten zur Parametrierung der Funktion des Implantats und zum anderen eine Gesamtheit von Programmobjekten aufweist, die zum Funktionieren des Programmierers in Beziehung mit den Daten der Parametrierung des Implantats notwendig sind,
- der Programmierer und das Implantat Einrichtungen aufweisen, die dazu geeignet sind, zwischen ihnen eine bidirektionale Verbindung zum Austausch von Informationen aufzubauen, die insbesondere den selektiven Transfer in Richtung des Programmierers von allen oder einem Teil der Daten und der Objekte des Implantats erlaubt, die in dem Programm des Programmierers fehlen,
- der Programmierer Einrichtungen zur Aktualisierung aufweist, die dazu geeignet sind, das Fernladen ausgehend von dem Implantat zumindest eines Teils der Objekte, die in dem Speicher dieses letzteren enthalten sind, zu steuern und diese Objekte denjenigen des Programms des Programmierers hinzuzufügen und/oder sie zu ersetzen.

2. Gesamtheit des Anpruchs 1, bei der die Einrichtung zur Aktualisierung des Programmiers aufweist:
- Einrichtungen, die dazu geeignet sind, durch Lesen des Speicher des Implantats eine Liste von Objekten aufzubauen, die sich dort mit ihrer jeweiligen Version befinden,
- Einrichtungen, die dazu geeignet sind, diese Liste mit derjenigen der Objekte des Programms des Programmierers zu vergleichen,
- Einrichtungen, die dazu geeignet sind, das Fernladen ausgehend von dem Implantat von Objekten, die nicht in dem Programm des Programmierers enthalten sind, zu steuern und diese Objekte dem Programm hinzuzufügen, und/oder
- Einrichtungen, die dazu geeignet sind, das Fernladen ausgehend von dem Implantat von Objekten zu steuern, die in dem Programm des Programmierers vorhanden sind, deren Version aber älter als diejenige ist, die sich in dem Implantat befindet, und diese Objekte in dem Programm zu ersetzen.

3. Gesamtheit gemäß Anspruch 1, bei der die Programmobjekte jeweils zumindest einen Objektidentifizierer, einen Versionsidentifizierer und einen Programmanweisungsblock aufweisen.

4. Implantat vom Typ einer aktiven implantierbaren medizinischen Vorrichtung, das dazu bestimmt ist mit einem Programmierer für die Gesamtheit gemäß Anspruch 1 zusammenzuwirken, wobei das Implantat dadurch gekennzeichnet ist, daß es aufweist:
- einen Speicher, der zum einen Daten zur Parametrierung der Funktion des Implantats und zum anderen eine Gesamtheit von Programmobjekten aufweist, die notwendig zum Funktionieren eines Programmierers in Beziehung mit den Daten zur Parametrierung des Implantats sind, wobei diese Objekte dazu geeignet sind, in Richtung des Programmierers gesandt zu werden, um das Programm dieses letzteren durch den selektiven Transfer in Richtung des Programmierers aller oder eines Teils der Daten und der Objekte, die in dem Programm des Programmierers fehlen, zu vervollständigen oder zu aktualisieren.

5. Implantat gemäß Anspruch 4, bei dem die Programmobjekte jeweils zumindest einen Objektidentifizierer, einen Versionsidentifizierer und einen Programmanweisungsblock aufweisen.

6. Externer Programmierer, der dazu bestimmt ist, mit dem Implantat vom Typ einer aktiven implantierbaren medizinischen Vorrichtung der Gesamtheit gemäß Anspruch 1 zusammenzuwirken, wobei der Programmierer dadurch gekennzeichnet ist, daß er aufweist:
- einen Speicher, der ein auf modulare Weise strukturiertes Programm vom objektorientierten Typ enthält, das aus einer Gesamtheit von Programmmodulen vom Objekttyp zusammengesetzt ist,
- Einrichtungen, die dazu geeignet sind, mit dem Implantat eine bidirektionale Verbindung zum Austausch von Informationen aufzubauen, die insbesondere den selektiven Transfer vom Implantat in Richtung des Programmierers von allen oder einem Teil der Daten zur Parametrierung der Funkion des Implantats und von Programmobjekten erlaubt, die notwendig zum Funktionieren eines Programmierers in Beziehung mit den Daten zur Parametrierung des Implantats sind, die in dem Programm des Programmierers fehlen, und
- Einrichtungen zur Aktualisierung, die dazu geeignet sind, das Fernladen ausgehend von dem Implantat zumindest eines Teiles der Objekte zu steuern, die in dem Speicher dieses letzteren enthalten sind, und diese Objekte denjenigen des Programmes des Programmierers hinzuzufügen und/oder sie zu substituieren.

7. Programmierer gemäß Anspruch 4, bei dem die Einrichtungen zur Aktualisierung aufweisen:
- Einrichtungen, die dazu geeignet sind, durch Lesen des Speichers des Implantats eine Liste von Objekten aufzubauen, die sich mit ihrer jeweiligen Version dort befinden,
- Einrichtungen, die dazu geeignet sind, diese Liste mit derjenigen der Objekte des Programmes des Programmierers zu vergleichen,
- Einrichtungen, die dazu geeignet sind, das Fernladen ausgehend von dem Implantat von Objekten, die nicht in dem Programm des Programmierers enthalten sind, zu steuern und diese Objekte dem Programm hinzuzufügen und/oder
- Einrichtungen, die dazu geeignet sind, das Fernladen von dem Implantat von Objekten, die in dem Programm des Programmierers enthalten sind, deren Version aber älter als diejenige ist, die sich in dem Implantat befindet, zu steuern und diese Objekte in dem Programm zu ersetzen.

8. Programmierer gemäß Anspruch 6, bei dem die Programmobjekte jeweils zumindest einen Objektidentifizierer, einen Versionsidentifizierer und einen Programmanweisungsblock aufweisen.

## Claims

1. A system comprising, firstly, an implant of the active implantable medical device type, and secondly, an external implant-programmer, the system being characterized in that:
. the programmer includes a memory containing modularly-structured software of the object-oriented type composed of a set of software modules of the object type;
. the implant includes a memory containing, firstly, parametric data associated with the functioning of the implant and, secondly, a set of software objects necessary for a programmer to function with said parametric data of the implant;
. the programmer and the implant include means that are capable of establishing a bi-directional link between them for exchanging information, making it possible, in particular, to transfer selectively all or some of said data and said objects which are missing in the programmer software, from the implant to the programmer; and
. the programmer includes updating means that are suitable for causing at least some of said objects contained in the memory of said implant to be downloaded from the implant and to be stored in the programmer software in addition to and/or as a replacement of the objects thereof.

2. The system of claim 1, in which the programmer updating means comprise:
. means that are capable, by reading the implant memory, of establishing a list of objects which are stored therein together with their respective versions;
. means that are capable of comparing said list with the list of the objects of the programmer software;
. means that are capable of controlling downloading, from the implant, of the objects that are absent from the programmer software, and of adding said objects to the software; and/or
. means that are capable of controlling downloading, from the implant, of the objects that are present in the programmer software but whose version is older than the version found in the implant, and of replacing said objects in the software.

3. A system according to claim 1, in which said software objects each comprise at least an object identifier, a version identifier, and a bloc of program instructions.

4. An implant of the active implantable medical device type, designed to cooperate with the programmer of the system according to claim 1, the implant being characterized in that it includes:
. a memory containing, firstly, parametric data associated with the functioning of the implant and, secondly, a set of software objects necessary for a programmer to function with said parametric data of the implant; said objects being suitable for being transmitted to the programmer in order to complete or update the software of said programmer by selectively transferring to the programmer all or some of said data and said objects which are missing in the programmer software.

5. The implant of claim 6, in which said software objects each comprise at least an object identifier, a version identifier, and a bloc of program instructions.

6. An external programmer, designed to cooperate with the implant of the active implantable medical device type of the system according to claim 1, the programmer being characterized in that it includes:
. a memory containing modularly-structured software of the object-oriented type composed of a set of software modules of the object type;
. means that are capable of establishing a bi-directional link for exchanging information with the implant, the link making it possible, in particular, to transfer selectively, from the implant to the programmer, all or some of the parametric data associated with the functioning of the implant, and of the software objects necessary for a programmer to function with said parametric data of the implant and which are missing in the programmer software; and
. updating means that are suitable for causing at least some of said objects contained in the memory of said implant to be downloaded from the implant and to be stored in the programmer software in addition to and/or as a replacement of the objects thereof.

7. The programmer of claim 4, in which the updating means comprise:
. means that are capable, by reading the implant memory, of establishing a list of objects which are stored therein together with their respective versions;
. means that are capable of comparing said list with the list of the objects of the programmer software;
. means that are capable of controlling downloading, from the implant, of the objects that are absent from the programmer software, and of adding said objects to the software; and/or
. means that are capable of controlling downloading, from the implant, of the objects that are present in the programmer software but whose version is older than the version found in the implant, and of replacing said objects in the software.

8. The programmer of claim 6, in which said software objects each comprise at least an object identifier, a version identifier, and a bloc of program instructions.
